# EUROPEAN PATENT SPECIFICATION

(11) **EP 1 558 299 B1**
(45) Date of publication and mention of the grant of the patent: **12.12.2012**
(21) Application number: 03773878.8
(22) Date of filing: 30.10.2003
(51) Int. Cl.: A61L 24/00, A61L 24/04

(54) **A POLYURETHANE BASED COMPOSITION FOR FILLING OR SHORT-CIRCUITING VASCULAR CAVITIES**
ZUSAMMENSETZUNG AUF BASIS VON POLYURETHAN ZUM FÜLLEN ODER KURZSCHLIESSEN VON VASKULÄREN KAVITÄTEN
COMPOSITION A BASE DE POLYURETHANE POUR REMPLIR OU METTRE EN COURT-CIRCUIT DES CAVITES VASCULAIRES

(30) Priority: 31.10.2002 HU 0203719
(43) Date of publication of application: 03.08.2005
(73) Proprietor: Hudák, István, 36 Pécs (HU); Doczi, Tamás, 7623 Pécs (HU)
(72) Inventor: HUDAK, István, H-36 Pécs (HU)
(74) Representative: Török, Ferenc
(86) International application number: PCT/HU2003/000090
(87) International publication number: WO 2004/039422

(56) References cited:
- EP-A- 0 280 451
- US-A- 6 017 977
- US-A1- 2001 004 710
- US-B1- 6 296 604

## Description

The invention is directed to the use of some special polyurethanes applied for filling or short-circuiting vascular cavities and also for manufacturing the relating compositions, moreover, the invention embraces the relating compositions and kits. In the invented use and compositions (kits) such polyurethane polymers play an essential role, which are soluble in biocompatible solvents or solvent mixtures and solidify following the removal of the solvent.

The composition according to the invention can be applied primarily for filling and occluding pathologically affected vessels in vascular malformations including aneurysms, with special regard to saccular aneurysms, and arterio-venous malformations (angioma) and arterio-venous fistulas (carotid-cavernous fistula) as well as vascularized tumors. The invention also concerns the therapeutic use of these compositions, since they provide the opportunity for treating the conditions related to the above-detailed phenomena by a new method.

Although the composition according to the invention can be applied for occlusion of intact blood vessels (thus providing for the short-circuiting of a pathologically affected/injured part of the vascular network) the forthcoming description will rather be devoted to its use for the treatment of vascular malformations, since this kind of problems occur much more frequently.

Vascular malformations can develop in any part of the vascular system. An advantageous feature of the invention is that the appropriate composition and method are applicable at any site of the body. Vascular malformations affecting the brain, however, are of special significance. Since the use of the composition and method according to the invention is especially effective in the case of vascular malformations affecting the brain, the further analysis will primarily concern the discussion of these, however, it is important to emphasize that the composition and method according to the invention can also be applied at any other site of the body.

The filling of the aneurysms on the vascular walls gains a special practical significance because the vascular walls weaken at the site of the aneurysms, thus creating a constant risk factor, owing to the increased probability of vascular ruptures. The possibly developing internal hemorrhage is especially dangerous within the brain (subarachnoidal hemorrhage).

The other essential group of conditions is related to the formation of the pathologically affected capillary system (arterio-venous malformation i.e. angioma). In these conditions, due to the shunt effect of the angioma, the main vessels are incapable of carrying sufficient blood, and, due to the rupture of the pathologically affected vessels, hemorrhages can easily develop. Pathological malformations also include the situation when the pathological vascular network is formed for the purposes of supplying a tumor (i.e. in the case of vascularized tumors). A positive therapeutic effect can also be expected in this case, provided that the pathologically affected vessels can be occluded.

The composition and method according to the invention are applicable in all the cases mentioned and discussed above.

According to a variety of the treatment of the above discussed types of conditions, the part of the space bordered by the walls of the vessels to be occluded needs to be filled with some suitable substance, thus carrying out the short-circuiting of the affected part. For the sake of simplicity, the composition and method according to the invention will be discussed here by way of analyzing aneurysms, emphasizing that the methods discussed are applicable in the cases of all similar malformations that can be managed by the occlusion of a part or a whole blood vessel or a whole vascular network. It also needs special emphasis that the composition and method according to the invention are effectively applicable in all the cases mentioned above, i.e. they should not be considered as specific for the management of aneurysms.

The occlusion of aneurysm is basically performed in two ways:
a) through trascranial operations, where on the neck providing for the communication between the pouch-like aneurysm and the parent vessel a metallic clip is fastened and, as a result, the aneurysm is short-circuited,
b) using an endovascular technique, where the vessels or the pouch of the aneurysm is filled with one or more substances.

The present invention falls under type b), so the following analysis will concern the details of the endovascular technique.
1. The balloon occlusion of the parent vessel is applicable in the cases when neither transcranial nor endovcascular selective final management of the aneurysm is possible. At the same time there is sufficient angiographic and clinical evidence that the function of the occluded artery can effectively be substituted by the collateral circulation. In some special cases it is possible to fill one aneurysm with one balloon (intrasaccular occlusion, for details see US 6 096 021, WO 98/09670).
2. The aneurysm can be filled with electrolytically detachable microspirals. This procedure is the currently applied standard method for the selective occlusion of aneurysms, where a microcatheter is inserted in the pouch of the aneurysm, which then is filled with microspirals.

Microspirals are made of extremely flexible, heat-treated platinum and come in different sizes. Thus the size most suitable for the given pouch can be selected. The microspiral is soldered to a steel conducting wire, with which it can be inserted into the cavity of the aneurysm through the microcatheter. If the positioning is favorable, the spiral can be removed from the conducting wire using different techniques depending on the type. As a rule, each aneurysm is occluded by several spirals. The insertion of the spirals is carried out until repeated angiography reveals the saturation of the contrast material in the pouch.

The limitations of the endovascular technique are as follows:
1. It is problematic if the neck of the pouch is too wide or the diameter of the aneurysm exceeds 25 mm (giant aneurysm).
2. The microspiral may get impacted in the cavity of the aneurysm i.e. the aneurysm reopens, so a relapse occurs.
3. In some cases the wall of the aneurysm may get perforated.
4. The cavity of the aneurysm may often be insufficiently filled by the microspirals, so full occlusion, i.e. the formation of the full thrombosis can only develop gradually, thus maintaining the risk of hemorrhage.
5. Sometimes the microspiral gets spontaneously separated from the steel conducting wire.
6. The insertion of the microspirals is getting more and more difficult with the increase in the number of spirals, as the previously inserted spirals prevent the new ones from being inserted.
7. The microcatheter may get removed from its ideal position due to its flexibility and volume. So, in some cases the catheter springs into the lumen of the parent vessel. In such cases the microcatheter needs to be repositioned.
8. On inserting each microspiral thromboembolism may develop, as the conducting wire needs to be inserted and, following the separation, it needs to be removed. The more spirals the aneurysm can take up, the longer is the operation and the more intense is the movement of the wire in the catheter, consequently the greater is the risk of thromboembolism.
9. A complete filling of the aneurysm with microspirals is only theoretical, since another microspiral can be inserted into a densely filled aneurysm pouch only in theory, so the filling of the cavity can never be a 100%, i.e. some space always remains unfilled in the network.

In the problematic cases described above the use of an additional microballoon may be necessary, which is filled opposite the neck of the pouch, thus forcing the spiral into a position which does not cause a stricture. The balloon is filled only for the time of spiral insertion and it is removed following the treatment. Similar is the function of a vascular stent placed in the parent vessel, which provides for a permanent fixation of the position of the spirals, and also influences the blood stream in the parent vessel, so it promotes a permanent thrombosis in the aneurysm.
(3) Another basic group of solutions to the problem includes those, which use a liquid substance for the filling of the vessel or the aneurysm, which solidifies later on. The invention falls under this category, so the next part of the discussion will concern the description of this kind of space-filling techniques. It needs to be noted that in certain cases methods 2 and 3 discussed above are combined (see US Patents 5,702,361, 6,017,977, 6,281,263 and 6,335,384).

The substance used for space-filling in all the solutions known to us is a polymer. These solutions can be divided into two further subgroups: a) the constituents of the polymer (its prepolymers) are delivered into the space to be filled, where *in situ* polymerization occurs, or b) the polymer is dissolved in a solvent (or it may be melted) and in this state it is delivered to the space to be filled, where, as a result of a gradual removal of the solvent (or cooling), the polymer re-solidifies. Method a) described above has not yielded any practically applicable result so far, and it is only mentioned here as a theoretically possible method of polymerization at the site of the space-filling. The present invention falls under method b) described above, which is based on dissolving polymers (here we mention that we have no information on practically applicable melting techniques), so the following part of this discussion will concern the description of such space-filling techniques. The subgroups below are set up on the basis of polymers used.
i) Cellulose acetate or diacetate based polymer (see e.g. US Patent 5,580,568).
   It needs to be emphasized that in US Patent 6,051,607 concentrating on this kind of polymers ethyl lactate is used as solvent. In the disclosure the attention is called that dimethyl sulphoxide (DMSO) frequently used in practice has a toxic effect on blood vessels [Chaloupka, Amer. Jour. Neur. Rad., 15: 1107 (1994)], while ethanol (e.g. 70% by volume) causes subarachnoidal bleeding [Sampei, K. at al., Interventional Neuroradiology, 38: 291-294 (1996)].
ii) Polymers containing albumin or compounds with a similar structure (e.g. in US Patent 5,894,022).
iii) Polyglycol acid based polymers (see EP 1,156,759).
iv) Acrylate based polymers (see EP 1,128,784 and US Patent 6,037,366).
v) Polyvinylalcohol based polymers (see e.g. WO 01/68720).
vi) Polyvinylacetate based polymers (see e.g. US Patents 5,925,683 and 6,160,025).
vii) Alginic acid based polymers (see US Patent 5,624,685).
viii) There are efforts in which polymers solidifying at body temperature are used. Such polymers include polyalkyloxide block polymers and some cellulose based compounds (for details see EP 1,148,895), and also some special N-substituted methacrylamid polymers (see US Patent 5,525,334).
ix) Ethylene-polyvinylalcohol (EVOH) co-polymers (see e.g. US patents 5,695,480, 5,667,767, 5,851,508 and 6,303,100).

From the above described solutions few have reached the clinical experimental phase. In the medical practice a composition based on N-butylcyanoacrylate is used occasionally (but it can only be applied in the case of pathological vessels related to tumors). In this field the most widely spread product is EVOH-based (with the brandname Onyx, Micro Therapeutics Inc., Irvine, CA). Although the application of this product covers a wider range (e.g. it can also be used in the case of vascular malformations in the brain), it also has a large number of disadvantages, including:
1) The density of the substance is too high for occluding the aneurysm, i.e. it can only be delivered through a thick catheter (0.6 mm - 1 mm).
2) Due to the high density an extremely high pressure has to be applied for delivery, which is extremely disadvantageous.
3) Due to the above mentioned reasons the injection takes too long, which in addition to the high density, is caused by the disadvantages described later on.
4) It cannot take up an ideal spherical shape for which embolizing substance may easily get into the parent vessel on the one hand, while on the other hand, the direction of the injected substance cannot be planned, therefore it needs to be injected in extremely small doses, and the previously injected dose needs to solidify before the following dose is injected. Due to this fact, the injecting procedure takes too long, and, in the case of a larger aneurysm, the operation may last for as long as 8-10 hours.
5) The catheter gets easily stuck to Onyx, which makes the separation extremely difficult, thus making the intervention risky.
6) Before the injection of Onyx the microcatheter needs to be washed with pure DMSO, which increases the risk of toxicity and, in addition, the dissolving procedure also requires a comparatively high amount of DMSO, which also increases the risk of toxicity.
7) The use of supplements (such stents, microspirals and balloons) is indispensable for Onyx to take up the appropriate shape of the aneurysm, which also complicates the operation.

As it was mentioned at the beginning of this description, some special polyurethane polymers play an essential part in the solution according to the present invention. Therefore the following description will concern the forms of utilizing polyurethane polymers in the fields covered by the present invention, and also in the adjoining fields.

In general, the commonly known polyurethanes are considered to be insoluble in any kind of solvents. Accordingly, the prior art techniques include only those in which the requirement of dissolving of the polyurethanes has not emerged.

So in some solutions including the use of balloons the recommended material of the balloon is polyurethane (for details see document WO 98/09670 mentioned above)

Some polyurethanes have thermoplastic features (i.e. they soften at high temperatures). Such substances are recommended for establishing a separable connection between the microspirals to be inserted into the aneurysm and the delivering catheter (see US Patents 5,911,737, 6,102,933 and 6,059,815).

US Patent 6,261,583 presents biologically decomposable thermoplastic implants. The general list of polymers includes the urethane-type polymers, however, in the concrete example poly(DL-lactide-co-glycolide) polymer is applied. No mention of the special area according to the present invention is made (where the use of biologically easily decomposing polymers is inappropriate).

The composition described in US Patent 6,296,604 contains a component sensitive to magnetic fields, which makes it possible for the polymers filling the target space to be directed to and kept in the appropriate site by means of an outside magnetic fields. In the general disclosure polyurethanes are also mentioned, however, concrete examples include only cyanoacrylate and cellulose acetate based polymers, i.e. no investigations into polyurethane polymers were carried out.

Related to the solvents contained in the compositions according to the present invention is US Patent 5,830,178, where the elimination of undesired side-effects caused by ethanol (EtOH) and the higher amounts of DMSO required by the commonly used procedures, is considered as a problem to be solved. The possible solution offered is that the concentration of these solvents (which are only used as alternatives, i.e. the authors do not raise the possibility of using a mixture of the two above mentioned solvents) in the blood cannot exceed a certain level, above which the constriction of the blood vessels and toxicological symptoms occur (the above prior art by Chaloupka and Shampei is used as reference).

A point in US Patent 5,702,361, in which polyurethanes are mentioned as polymers whose pre-polymers are liquid (consequently polyurethanes are regarded as solid substances), refers to the insolubility of polyurethanes. The description contains no reference of any possibility for polyurethanes to become liquid by way of dissolving (cellulose diacetate and EVOH polymers are discussed in detail).

Finally, US Patent 6,342,202 and the application published under US 2001/0024637 (they belong to the same family of patents, as both of them originate from US applications Nos. 08/665822 and 08/802252), in which similar compositions are presented, and in the general instructions (for details see e.g. the abstract), polyurethanes are mentioned, however, in the list given in the claims polyurethanes are not present. This fact is related to the experimental results presented in Table 1 of the above documents, which suggest that polyurethanes do not dissolve in DMSO, consequently it is not suitable for using in the field of the invention (for details see the paragraph following Table 1). In accordance with this finding, the other easily soluble polymers are claimed in the use claims.

Summing up the above discussion, it is clear that prior art contains no reference according to which polyurethane based compositions could be used for occluding aneurysms and blood vessels, on the other hand there exists a teaching (and it can be found in a document originating from a company recognized in the field), according to which polyurethanes are impossible to use for these purposes.

In the course of investigating into substances to be used for occluding or short-circuiting aneurysms and blood vessels, surprisingly we have found that certain polyurethanes can be used for producing a composition showing excellent features and applicability for occluding or short-circuiting vascular cavities. The implementation of the invention is feasible by using special polyurethanes, which are soluble in solvents or solvent mixtures. The expectations for the applied solvent or solvent mixture is that it needs to mingle with body fluids, primarily with blood, and so the solvent (or part of it) needs to leave the dissolved polymer delivered to the site of final utilization, and thus the polyurethane can re-solidify there.

In line with the above discussion, the first aspect of the present invention is the use of a polyurethane in manufacturing of a composition for filling or short-circuiting vascular cavities, where the composition consists essentially of of polyurethane dissolved in DMSO or C2-C4 alcohol or in a mixture of them, optionally together with one or more auxiliary material(s).

A further aspect of the invention is a composition for use in filling or short-circuiting vascular cavities, comprising polyurethane dissolved in DMSO or C2-C4 alcohol or in a mixture of them, consisting essentially of polyurethane dissolved in DMSO or C2-C4 alcohol or in a mixture of them, optionally together with one or more auxiliary material(s).

An advantegous application of the invention is embodied in a medical kit containing the individual components separated. The kit can be used for filling or short-circuiting vascular cavities, where the medical kit consists essentially of the following components which are formulated separately or some of them are formulated in a common subunit:
a) a polyurethane which is soluble in DMSO or C2-C4 alcohol or in a mixture of them,
b) DMSO or C2-C4 alcohol or a mixture of them,
c) optionally one or more auxiliary material(s).

The substances and methods required for the implementation of the invention are described below in detail:

### 1. Polyurethane

As has already been mentioned, polyurethanes usually do not dissolve in commonly known solvents and they also show a high resistance against aggressive (destructive) fluids. Therefore a specific group of polyurethanes includes those, which can be used according to the present invention, since the requirement towards them is that they can be dissolved in a solvent being applicable in human or animal organisms, however, they do not dissolve in body fluids (primarily in the blood). Consequently, the polyurethane used gradually solidifies, while the solvent dissolves into the blood (i.e. the solvent needs to dissolve in body fluids, primarily in the blood).

The usability of a polyurethane in the present invention is determined by the fact whether a solution of the required viscosity can be prepared from it by dissolving it in a suitable solvent. Due to the technique used (the dissolved polymer is delivered to the destination through a catheter where it is supposed to fill up the part of space and it is not supposed to dislocated afterwards) neither too thin (low viscosity) nor too thick (high viscosity) solution can be used.

It is worth noting that in various areas of use (filling saccular aneurisms in the brain or in the case of occluding the affected blood vessels in arteriovenous malformations or vascularized tumors) the application of solutions with different viscosity is advisable. It can be said in general that the viscosity of the polyurethane solution should be less than 5000 mPa.s, preferably 10-3500 mPa.s, even more preferably 5-1000 mPa.s at 23 °C, where the thicker solution (with a viscosity higher than 150 mPa.s, preferably higher than 250 mPa.s, even more preferably 200-400 mPa.s at 23 °C) is used for filling aneurisms, while in the case of angiomas and vascularized tumors the use of the thinner solution is advisable (with a viscosity lower than 1000 mPa.s, preferably lower than 250 mPa.s, even more preferably 5-50 mPa.s at 23 °C).

Generally speaking, polyurethanes are polymers obtained by polyadditional reactions of diols and/or triols and isocyanates and/or triisocyanates. A common feature is that their molecules contain urethane groups (R¹-NH-CO-O-R²). Apart from the simplest cases, their structure is impossible to describe accurately due to the variant running of the polymerization reactions of the monomers used, and also to the fact that sometimes several kinds of monomers are used during production even within the same monomer type. For the purposes of the invention, the use of polyurethanes based on diols and diisocyanates is advantageous, as it is easier to produce soluble polyurethanes from them.

Examples to suitable diols are as follows: ethylene glycol, 1,2-propylene glycol, 1,3-propylene glycol, 1,4-butylene glycol, 2,3-butylene glycol, 1,6-hexandiol, 1,8-octanediol, neopentylglycol, 1,4-bis(hydroxymethyl)cyclohexane, 2-methyl-1,3-propandiol, diethylene glycol, triethylene glycol, tetraethylene glycol and polyethylene glycols with higher molecular weight, dipropylene glycol and polypropylene glycols, and also dibutylene glycol and polybutylene glycols and other oxyalkylene glycols.

Preferred starting monomers are diols characterized by the formula HO-R¹-OH (alkylenediols or alkylenediol monomers), where R¹ is typically C₁-C₈ (i.e. having 1 to 8 carbon atoms) alkylene group (alkanediyl group), preferably -(CH₂)₃-, -(CH₂)-, -(CH₂)₂-CH(CH₃)- group. Highly preferred is the - (CH₂)₄- group (1,4-buthandiol is the corresponding dialcohol, named also as 1,4-butylene glycol).

It is known for a skilled person that the above-mentioned ether-type diols (polyethylene glycols, polypropylene glycols and polybutylene glycols) constitute a special sub-group of the diols which can be formed by condensation of the alkylenediols with elimination of water [see RÖMPPS Chemie-Lexikon, 7th Edition, Kosmos-Verlag, Stuttgart, title: "Polyurethane"]. These compounds can be regarded as "polymerization" product of the alkylenediols and can be described by the general formula HO-(R¹-O)ₙ-H, wherein R¹ has the above-detailed meaning and n is 2 to 40, preferably 5 to 20, more preferably 8 to 18. Because the 1,4-butylene glycol (1,4-butanediol) can be obtained by ring-opening of tetrahydrofurane, the ether-type diols having -(CH₂)₄- group in the meaning of R¹ are often called as "polytetrahydrofurane".

A preferred subgroup of the polyurethane usable in the present invention embraces such polyurethanes in which a remarkable part of the alkylenediols (up to 99 % by weight, preferably 50 to 95 % by weight, more preferably 70 to 90 %) is in the above-detailed "polymerized" form (it is better to use the phrase "oligomerized" when n is 3 to 10). These molecular parts make the polymer softer and more flexible.

In the preferred embodiments and examples given below the alkylenediol monomer is referred to generally, but it is underlined that a preferred polyurethane contains the above-detailed ether-type polymeric (oligomeric) parts built up from the alkylenediol monomers in a remarkable amount, which parts have "polytetrahydrofurane" structure in a more preferred embodiment.

It is worth noting that in the case of the above classification of polymers [for details see points i-ix)] the majority of the patent descriptions referred to includes an extremely wide range of theoretically usable polymers. These patents were classified by us with regard to the types of polymers for which they provided teaching (this can primarily be decided on the basis of the examples). In several patents referred above certain urethane/carbonate co-polymers are mentioned. In such polymers, however, a polycarbonate type of polymer is used as diol, so the structure of these compounds is obviously far from the polyurethanes discussed above. (These compounds constitute a transition between polycarbonates and polyurethanes.) It also needs to be emphasized that we have found no such solution where this polymer type had been used.

A preferred group of the other types of starting monomers includes diisocyanates, which can be characterized by the general formula O=C=N-R²-N=C=O.

Examples to suitable diisocyanates are as follows: 2,4- or 2,6- toluylene-diisocyanate (TDI) (i.e. toluene-2,4-diisocyanate and toluene-2,6-diisocyanate), m-phenylene-diisocyanate, hexamethylene-1,6-diisocyanate, tetramethylene-1,4- diisocyanate, cyclohexane-1,4-diisocyanate, hexahydrotoluene-2,4-diisocyanate, hexahydrotoluene-2,6-diisocyanate, naphtaline-1,5-diisocyanate, diphenyl-methane-4,4'-diisocyanate (MDI, i.e.: methylene-biphenyl-diisocyanate), 4,4'-diphenylene-diisocyanate, 3,3'-dimethoxi-4,4'-biphenyl-diisocyanate, 3,3'-dimethyl-4,4'-biphenil-diisocyanate and 3,3'-dimethyl-diphenyl-methane-4,4'-diisocyanate. Even more preferred compounds as follows: 2,4 or 2,6 toluylene-diisocyanate (TDI), 1,6-hexane-diisocyanate and diphenyl-methane-4,4'-diisocyanate (MDI). Especially preferred is diphenyl-methane-4,4'-diisocyanate (MDI).

As it has been mentioned, there can be polyurethane polymers containing several types of diols or several types of diisocyanates, and certain quantities of monomers containing three functional groups are also used for ensuring spatial polymerization (curing).

Without binding ourselves to the theory discussed below, it seems that the solubility of polyurethanes is acceptable if the polymer chains do not branch (they are linear), as the development of spatial polymerization (curing) obviously counteracts solubility. The solubility of the suitable polymers with linear chain obviously depends on the average molecular weight of the polymer, with special regard to the concrete distribution of the molecular weight. The selection of the polyurethane with the suitable chain structure and molecular weight (distribution of molecular weight), i.e. the selection of the polyurethane with the suitable features of dissolving, is considered as an obvious routine task for the specialist.

Here we would mention that, for obtaining the desired molecular weight, often a so-called "chain-closing" compound (e.g. caprolactam) is applied during the preparation of the polyurethane to avoid the formation of polymer molecules having undesired high molecular weight. The preferred amount of this component is 0.01 to 5, more preferably 0.1 to 1, further more preferably 0.2 to 0.5 % by weight.

We found especially advantageous the polyurethane constituted by 1,4-buthanediol and diphenylmethane-4,4'-diisocyanate (MDI) monomers, whose average molecular weight is 4 000-70 000 Dalton and even more preferably 20 000-35 000 Dalton. For example the commercially available polyurethane with the trade name TECOTHANE (TECOTHANE TT-1065D, Thermedix Polymer Products, USA) proved to be suitable. As it was discussed above, a remarkable part or all of the alkylenediol monomers are coupled to each other to form polyether-type diol in the preferred embodiments. Of course, in such cases the alkylenediol monomer/isocyanate monomer ratio is bigger than 1, for example 2 to 8, preferably 3 to 6.

Here we would note that the polyurethane molecular weights mentioned in the specification are determined by viscosimetry which gives molecular weight values being between the number average molecular weight (Mₙ) and the weight average molecular weight (M_{w}) (closer to weight average molecular weight).

The definition of the types of the average molecular weights and the discussion of the molecular weight determinations can be found e.g. in Billmeyer, F.W.: Textbook of Polymer Science, J. Wiley, New York, 1984; Cowie, J.M.G.: Chemistry and Physics of Modem Materials, Blackie and Son, London, 1991; Elias, H.-G.: Macromolecules, John Wiley, London, 1977.

### 2. Solvent

The above discussion suggests that the solvent usable in the procedure according to the invention must have the following features:
- it needs to be usable in human or animal organisms (it must not be toxic in the applied concentration);
- it needs to dissolve the polyurethane used by itself or mixed with an other solvent; .
- it needs to be soluble in body fluids, primarily in blood, so it needs to be capable of being discharged from the dissolved polymer into the body fluid (primarily into the blood).

The solvents to be considered in the field include the following: dimethyl sulphoxide (DMSO), C2-C4 alcohols, e.g. ethyl alcohol (EtOH) or propyl alcohol, ethyl lactate, dimethyl formamide.

We found DMSO and EtOH advantageous and their mixture especially advantageous, preferably in 1:10-10:1 volume ratio, and even more preferably in 1:3-3:1 volume ratio. In practice the 1:1 ratio mixture of DMSO and EtOH gave very good results, where DMSO of analytical purity and 96% by volume of EtOH was used, however it is even more preferred to use absolute ethanol (minimum 99.2 % by volume of EtOH content, cf: Martindale, The complete drug reference 32nd edition, USP XXII, Dehydrated Alcohol).

DMSO is known to solve well in body fluids, including the blood, and it gets eliminated from the organism comparatively fast. Another essential discovery of ours is that it is capable of dissolving some polyurethanes. C2-C4 alcohols, including EtOH, are commonly known to dissolve well in body fluids, including the blood, and they are excreted from the organism fast. However, the alcohols mentioned above are not capable of dissolving polyurethane types of polymers, while in the mixture of DMSO and alcohol some polyurethanes can be dissolved. A curious and practically very important advantage follows from the use of such a solvent mixture, preferably the mixture of DMSO/EtOH, as in the process of solidifying, the majority of EtOH does not get out into the body fluids since it accumulates in the inclusions of the solidified polyurethanes (i.e. only DMSO discharges into the blood). An obvious advantage of this case is that in this way less DMSO gets into the organism and the formed polymer becomes soft and spongy in structure, thus causing no wall ruptures in the aneurisms.

### 3. Auxiliary materials

The solution with the polyurethane content to be delivered to the site of the intervention may contain one or more auxiliary materials commonly used in the field. Of these the most important are the contrast materials. The role of contrast materials is to make it possible to follow the movement of the polyurethane solution within the body visually (e.g. on a screen). Radiating contrast materials (e.g. isotopes) or paramagnetic contrast materials can also be used, however in practice the most important are those contrast materials, which absorb radiation to a great extent, so the movement of the polyurethane solution within the body can be visualized by way of "X-raying" the examined part of the body with appropriate radiation. For this purpose materials containing tantalum, iodine, barium, tungsten and bismuth can be used. Of these preferred are tantalum micronized powder, tantalum oxide, barium sulphate, ethyl-10(p-iodinephenyl)undecylate and tungsten and especially preferred is tantalum micronized powder (average particle size: 1-10 µm).

### 4. Body fluids

By the term body fluids we mean liquids with water content occurring in human or animal organisms, so they are: blood, liquor, cerebral and spinal fluid, lymph and urine.

### 5. Catheter

The dissolved polyurethane can be delivered to the site of the intervention through a flexible catheter. We can use those commonly applied in the field e.g. the products of the following manufacturers: Boston Scientific, Micro Therapeutics, Cordis (USA manufacturers). These catheters are especially usable in the case of blood vessel occlusions, while in the case of the filling of aneurisms - when it is necessary for the polymer to take up a spherical shape - it is advisable to shape the end of the catheter so that it can help the development of the spherical shape. Thus in such cases it is advisable to round off the edge of the tube at the end of the catheter and/or promote the adhesion of the polyurethane in some other way.

The preferred size of the catheter used is 0.1-1.0 mm. The preferred diameter of the catheter also depends on the viscosity of the polyurethane solution used.

### 6. Compositions and kits

The object of the invention includes the composition for direct use to fill up or to short-circuit the vascular cavities, and also the medical kit containing the individual components presented separately, from which the polyurethane solution to be injected can be prepared. Such a kit can contain each component presented separately or in a way where the individual components are already mixed. Thus the kit may contain the polyurethane, preferably in powder form, the solvent (or its constituents separated) and the auxiliary materials separated. It is advisable, however, separately present a) the polyurethane in powdered form, b) the final solvent mixture and c) the auxiliary materials, and to compile the kit of these. As the polyurethane constitutes a stable solution with several solvents, that formulation is advantageous which already contains the polyurethane solution with the required viscosity. Provided that we use auxiliary materials and that they are added to the polyurethane solution, the final composition is produced, while if the auxiliary materials are presented separately then the composition is presented as a kit.

We also found another formulation being advantageous, in which the tantalum powder used as an auxiliary material is contained in the solvent mixture, as in this case the unwanted large particles of the tantalum powder can easily be separated, and the polyurethane is presented in granulated form in a separate containers.

The kit preferably contains a description of the preparation and the utilization of the composition.

It is worth mentioning that the composition according to the invention is prepared by simply mixing the components using usual equipment and techniques. The medical utilization of the composition, as it can be seen in the following examples, is carried out by means of the techniques commonly known and used by a specialist. Furthermore, the compositions and kits according to the invention are preferably sterilized in usual way, e.g. by heat or radiation sterilization.

### UTILITY

The usability of the invention is supported by the following summary of the advantageous features:
1) The material takes up a spherical shape immediately after the injection, and, following further administration, being in contact with the wall of the aneurysm, takes up its shape.
2) It is extremely plastic and soft which highly promotes the prevention ofruptures. Its softness derives from the fact that it starts solidifying from the surface, thus it is only a very thin layer that solidifies first, which, however, happens immediately after it gets into contact with the blood and thus the space-filling form stabilizes, which prevents the rupture. The inner part of the material solidifies only gradually proceeding inwards from layer to layer for more than two hours.
3) The slow solidifying process is especially advantageous, as the solvent used diffuses from the material into the blood only very slowly, thus causing no toxicity in the cells.
4) The slow solidifying process is also advantageous because for one hour after an incidental overdosing, by means of an instrument (balloon or stent), the material can be shaped so that the circulation is maintained in the parent vessel. The other possibility is that in the case of overdosing low quantities can be withdrawn through the catheter.
5) The microcatheter (unlike the microspiral) does not get dislocated from the cavity of the aneurysm on injecting the polymer, even it is in unstable position.
6) The material can be made contrastive by adding the commonly used auxiliary materials such as tantalum micronized powder or materials containing iodine, barium, tungsten and bismuth, so the quantity of the injected material can easily be controlled.

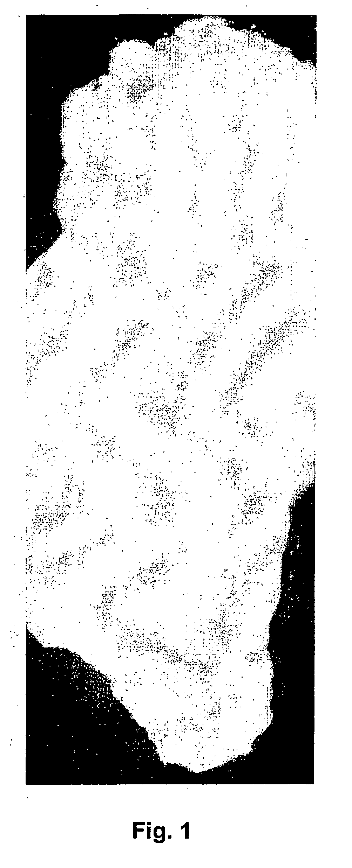

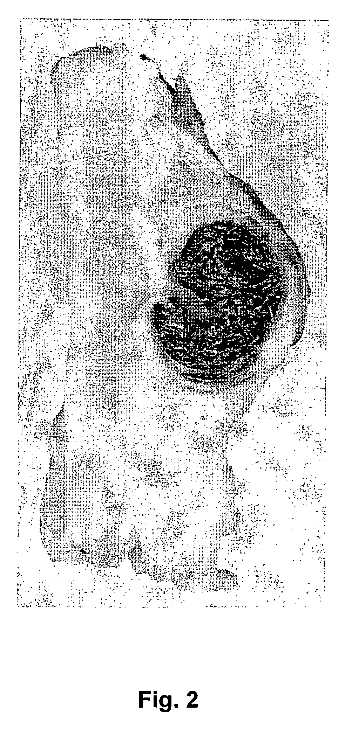

However, if a monitoring technique other than X-ray is used (isotopic, MNR, ultrasound, or endoscopic observation), either other contrast materials (e.g. radiating isotopes, paramagnetic materials) or no contrast materials need to be used.
7) The liquidity of the substance makes possible the use of catheter with an inner diameter of 0.1 mm (cf. in the case of using ONYX a catheter with a diameter of 0.8 mm is used).
8) The substance does not solidify in the catheter (observations prove that it does not solidify even after a day), which is important because, if needed, further amounts can be administered for achieving the occlusion without causing any delay due to the solidification in the catheter.
9) In the case of a most advantageous implementation, where in addition to DMSO a further alcohol-type biocompatible solvent (preferably EtOH) is used, in our experience the majority of this second alcohol-type solvent remains in the substance, which results in further decreasing the total amount and concentration of DMSO discharged into the blood flow.
10) Another outstanding characteristic feature of the substance is that on being injected into the aneurysm, despite the turbulence, it remains at the end of the catheter, i.e. it is not swept away with the blood flow. This makes the intervention a lot easier because further tools (further catheters, balloon catheters, stents, GDCs) are rarely necessary.
11) As the separation of the substance from the catheter is easy, no coaxial catheter is necessary.
12) The polyurethane is a polymer that can be decomposed biologically, however this process is time consuming and slow (which ensures the time for the formation of the intima). Therefore if a big aneurysm is occluded, then, due to this characteristic feature, the volume of the polymer injected decreases as time passes (thus the part of the space filled decreases, which can be advantageous as the pressure it exerted on the environment decreases), however, the new intima (the inner wall of the vessel) forms at the neck of the aneurysm before this takes place (see Figure 1).
13) In its solid state the polyurethane is spongy, i.e. microporous, into which collagen can grow into (see Figure 2), thus making the migration in the thrombus impossible postoperatively, therefore no recurrence is likely.
14) The injection using the catheter takes a very short time, only a few seconds, and the catheter can be separated immediately after the injection, i.e. the polymerization does not need to be awaited.
15) The substance does not stick to the catheter or to the wall of the vessel.
16) It is biocompatible and it is not pyrogenic.
17) In the course of solidification it does not warm up.
18) The catheter does not need to be rinsed with a thinning agent prior to injecting the substance, i.e. it can be injected into the catheter immediately. This is an essential practical advantage compared to the techniques known so far, as due to the necessity of rinsing (on the insertion of the catheter the polymer solution to be injected must not be in the catheter) further significant amounts of DMSO get into the blood circulation. Due to the toxic effect of DMSO, this kind of rinsing can only be performed slowly in the case of the known techniques, thus the elimination of this rinsing procedure results in further significant decrease in the duration of the intervention.
19) There is no contraindication concerning the size of the aneurysm, this substance can be used in the case of any size.
20) It is of universal use (usable for occluding aneurysms, blood vessels and tumors).
21) It can be used in the case of aneurysms and AVMs of any size.

The further discussion will concern the presentation of concrete embodiments of the solution according to the invention. The procedures described can obviously be modified by specialists without moving away from the idea of the invention and without getting out of the scope of protection.

It is noted that sterile 96 % by volume of ethanol was applied as concentrated ethanol and a sterile, non-pyrogenic and endotoxin-free DMSO marketed by WAK-CHEMIE MEDICAL GmBH was used in the experiments.

### Example 1

### Occluding experimental saccular aneurysms endovascularly, using liquid embolizing substance

### A) Producing the experimental aneurysms

A procedure generally accepted and published in the international literature (Chaloupka J C at al: American Journal ofNeuroloradiology, 20: 401-410, 199) will be presented shortly below.

Six dogs (altogether with 10 aneurysms) were included in the animal experiment. Permission for the total procedure of the intervention was granted by the Ethical Standing Committee of the University of Kaposvár, Hungary. The procedure was carried out in accordance with the internationally used ethical requirements for animal experiments. The age of the animals ranged between half a year and one year, they weighed between 30 and 40 kg, both males and females, and were kept on standard laboratory diet. They consumed no food during the night preceding the intervention and premedication prior to the operation was carried out by administering Ketamine (20 mg/kg) intramuscularly. The general anesthesia was maintained by mechanical breathing and Propofol was administered intravenously. Ten aneurysms in the six animals were prepared by two neurosurgeons, using microsurgical methods. The aneurysms on the common carotic artery of the dog were prepared in accordance with the description found in the international literature (Murayana Y et. al. American Journal of Neuroradiology, 21: 1726-1735, 2000), using microsurgical techniques. Two aneurysms with a diameter of 8-10 mm and with a neck size of 4-8 mm were prepared. For this, the right jugular external vein was isolated and after separating it into two equal segments they were excised, which were used for preparing the aneurysm-sac. An arteriotomy of 4-8 mm of length was prepared on the common carotic artery and an "end to side" anastomosis was prepared using 8-0 prolen thread knotted stitches. The height of the aneurysm was tailored to 8-10 mm by ligating the vein grafts.

After the preparation of the aneurysm, angiography was used to detect the exact morphology of the aneurysm. Both pre-embolisational and post-embolisational angiograms were made.

### B) Producing the embolizing substance

10 g of polyurethane polymer, built up from 1,4-butanediol and diphenylmethane-4,4'-diisocyanate (MDI) monomers [the average molecular weight is 29 000 Dalton, where the molar ratio of 1,4-butanediol (alkylenediol monomer) and MDI is 5.8:1, and 87 % of the 1,4-butanediol is in "polytetrahydrofurane" form], is dissolved in the mixture of 20 ml of DMSO and 28 ml of concentrated ethyl alcohol (EtOH) according to the following: a) first the polyurethane is dissolved in 20 ml of DMSO for 24 hours, b) then concentrated ethyl alcohol is administered in 0.2-0.5 ml doses, while the solution is mixed by rotation. The solution process is performed in a sealed system with the exclusion of moisture. The viscosity of the resulting solution is 287.1 mPa.s (cP) at 23 °C, while its density is 0.943 g/cm³ at 23 °C. Before utilization, 20 g of micronized tantalum powder is added to the solution.

### C) Delivering the embolizing substance

Using Seldinger's method a catheter was inserted into the right femoral artery (5F), the end of the catheter was in the common carotic artery. This was confirmed by making control angiography, which allowed for visualizing and checking the suitability of the aneurysm prepared an hour before. A microcatheter (inner diameter of 0.5 mm, outer diameter of 0.6 mm) was inserted into the lumen of the aneurysm through the 5F catheter. Super selective angiography was performed by adding 0.5 mm of contrast material (Ultravist). The inner shape and suitability of the aneurysm was checked again.

The injecting of the embolizing substance was started without any preliminary rinsing, using a special syringe with a threaded plunger, which allows a highly accurate qualitative control.

The lumen of the aneurysm was gradually filled with the embolizing substance. The progression of the procedure up till the total filling of the lumen was controlled by the road-map method, a continuous X-ray subtraction technique (Siemmens DSA). Then the inner cavity of the aneurysm was fully filled by the substance, while it did not protrude into the lumen of the parent vessel, neither did it discharge any debris of the embolizing substance into it. Control angiography was used to confirm the lack of blood circulation in the aneurysm and the intact blood flow in the parent vessel. Right after that the microcatheter was separated.

The whole procedure took 45 minutes.

Within 30 minutes another control angiography was carried out on the carotis to exclude any possible intracranial embolization in the vessels. No such complication has been detected in any of the cases. Each dog was asymptomatic after awaking.

Histopathological analysis revealed no toxicity in the tissues. The occlusion of the vessels proved to be lasting, after six months the polyurethane got organized and normal intima formed over it.

The most characteristic pictures taken during the implementation are presented below (see Figures 1-3).
Figure 1 is a picture of a preparation i.e. an excision of a carotic artery following a one-month control angiography. The segment of the carotic internal artery containing the aneurysm is opened longitudinally opposite the neck of the aneurysm and it is spread. At the neck of the aneurysm a fine dent can be seen, which is dark in color and is covered by an endothelium (intima) with a flat surface. The occlusion of the aneurysm is successful, which can be confirmed by the fact that the earlier neck of the aneurysm is occluded by the intima of the artery.
Figure 2 is a picture of a preparation of a one- month postoperative control, which cuts through the wall of the internal carotic artery saggitally (in saggitalis direction) as well as the neck of the aneurysm and the aneurysm-sac. It can be seen that the aneurysm is fully occluded, and after a month, in addition to the total organization collagen fibers grow into the polyurethane, which totally occludes the cavity of the aneurysm. It can also be seen that the earlier neck of the aneurysm is bridged at the new neck by a flat-surfaced intima. The network of the fibrin fibers grew into the spongy structure of the polyurethane similarly to trabecules.

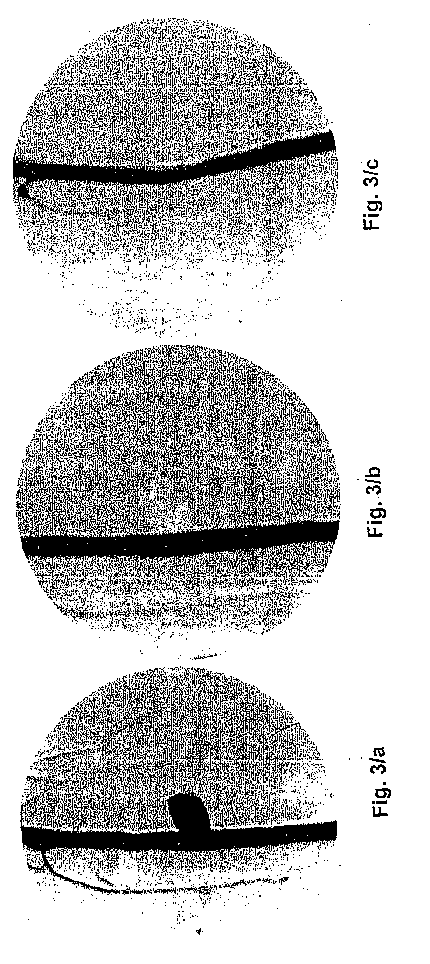

In Figure 3 there are three pictures (3/a, 3/b and 3/c).
Picture 3/a: The common carotic artery of a dog filled with contrast material, with a wide necked and large size aneurysm (the depth of the aneurysm is 2.5 times as big as that of the carotic artery, while the neck diameter of the aneurysm is twice as long as that of the carotic artery).
Picture 3/b (in the middle): acute control angiography carried out following the postoperative delivery of the polyurethane into the aneurysm and the short-circuiting of the aneurysm. It can be seen that no blood invades the aneurysm and the carotis wall is straight and sharply delineates.
Picture 3/c angiography performed one-month following the operation: the aneurysm does not fill, the wall of the carotic artery delineates sharply, it is normal. It is a successful operation.

### Example 2

### Modeling the treatment of arteriovenous malformations (angioma) and vascularized (very rich in blood vessels) tumors.

### A) Preparation

Similarly to the experimental conditions described in Example 1 the renal artery was catheterized in three dogs and angiography was performed using 5 ml of Ultravist contrast material. The vascular system of the right kidney was visualized.

### B) The production of the embolizing substance to be used.

10 g of polyurethane polymer built up from 1,4-butanediol and diphenylmethane-4,4'-diisocyanate (MDI) monomers (see Example 1) is dissolved in the mixture of 27 ml of DMSO and 36 ml of concentrated ethyl alcohol (EtOH). The viscosity of the resulting solution is 11.1 mPa.s (cP) at 23 °C, while its density is 0.925 g/cm³ at 23 °C. Before utilization, 20 g of micronized tantalum powder is added to the solution.

### C) The delivery of the embolizing substance

Similarly to the method described in Example 1, 0.5 ml of embolizing substance is delivered into the catheter, then the right renal artery was occluded by the embolizing material. In the course of the experiment control angiography was performed on several occasions to check if the renal vessels are fully occluded.

One hour later the kidneys were removed and they were analyzed histologically. The successful occlusion of the vessels could be confirmed.

### Example 3

The experiment described in Example 1 was performed apart from the difference that the 10 g of polyurethane was dissolved in 43 ml of pure DMSO thus achieving the polyurethane solution of the required viscosity. The aneurysm was short-circuited successfully using exactly the same method as described in Example 1.

### Example 4

The experiment described in Example 2 was performed with the difference that the 10g of polyurethane was dissolved in 60 ml of pure DMSO, thus achieving the polyurethane solution of the required viscosity. The occlusion of the vessels was successfully carried out using the same method.

### Example 5

The experiment described in Example 1 was carried out with the difference that the 5 g of polyurethane marketed under the commercial name TECOTHANE TT-1065D (Thermedich Polymer Products, USA) was dissolved in 43 ml of DMSO [viscosity: 3943.8 mPa.s (24.5 °C), density: 1.002 g/m³ (24.5 °C)]. The aneurysm was also successfully short-circuited, although, due to the higher degree of viscosity, it proved to be more difficult from practical point of view.

### Example 6

The experiment described in Example 2 was carried out with the difference that the 5 g of polyurethane marketed under the commercial name TECOTHANE TT-1065D was dissolved in the mixture of 27 ml of DMSO and 36 ml of concentrated ethyl alcohol [viscosity: 738.1 mPa.s (24.5 °C), density: 0.943 g/m³ (24.5 °C)]. The vessels of the right kidney were similarly successfully occluded.

### Formulation Example 1

The following substances are filled in glass ampoules then the ampoules are closed hermetically.
Ampoule 1: 10 mg of polyurethane granulate.
Ampoule 2: a mixture of 30 ml of DMSO and 30 ml of EtOH.
Ampoule 3: 20 mg of micronized tantalum powder.

The ampoules are put into a container shaped up for the proper fixation of the ampoules. In the container the instruction of use is also included.

### Formulation Example 2

The following substances are filled in PVC then the ampoules are hermetically closed.
Ampoule 1: 15 mg polyurethane granulate dissolved in a mixture and 40 ml of DMSO and 45 ml of EtOH.
Ampoule 2: a mixture of 30 ml of micronized tantalum powder.

The ampoules are put into a container shaped up for the proper fixation of the ampoules. In the container the instruction of use is also included.

### Formulation Example 3

10 g of polyurethane granulate was dissolved in the mixture of 25 ml DMSO and 25 ml of EtOH, then it is filled into a glass ampoule, into which 20 mg of micronized tantalum powder was filled previously. The glass ampoule is hermetically closed.

### Formulation Example 4

The following substances are filled into glass ampoules and the ampoules are hermetically closed.
Ampoule 1: 15 mg of polyurethane granulate.
Ampoule 2: 30 ml of micronized tantalum powder and 90 ml of DMSO.

The ampoules are put into a container shaped up for the proper fixation of the ampoules. In the container the instruction of use is also included.

## Claims

1. Use of a polyurethane in manufacturing of a composition for filling or short-circuiting vascular cavities, where the composition consists essentially of polyurethane dissolved in DMSO or C2-C4 alcohol or in a mixture of them, optionally together with one or more auxiliary material(s).

2. Composition for use in filling or short-circuiting vascular cavities, consisting essentially of polyurethane dissolved in DMSO or C2-C4 alcohol or in a mixture of them, optionally together with one or more auxiliary material(s).

3. The use or composition according to any of claims 1 or 2, where mixture of DMSO and EtOH is applied in a volume ratio of 1:10 - 10:1, preferably of 1:3 - 3:1.

4. The use or composition according to any of claims 1 to 3, where the auxiliary material is a contrast material selected from the following group: a substance containing tantalum, iodine, barium, tungsten and/or bismuth.

5. The use or composition according to any of claims 1 to 4, where the molecular mass of the polyurethane is 4000 to 70,000 Dalton, preferably 20,000 to 35,000 Dalton.

6. Use of a polyurethane in manufacturing of a medical kit for filling or short-circuiting vascular cavities, where the medical kit consists essentially of the following components which are formulated separately or some of them are formulated in a common subunit:
a) a polyurethane which is soluble in DMSO or C2-C4 alcohol or in a mixture of them,
b) DMSO or C2-C4 alcohol or a mixture of them,
c) optionally one or more auxiliary material(s).

7. A medical kit for filling or short-circuiting vascular cavities, consisting essentially of the following components which are formulated separately or some of them are formulated in a common subunit:
a) a polyurethane which is soluble in DMSO or C2-C4 alcohol or in a mixture of them,
b) DMSO or C2-C4 or a mixture of them,
c) optionally one or more auxiliary material(s).

8. The use or medical kit according to any of claims 6 or 7, where mixture of DMSO and EtOH is applied in a volume ratio of 1:10 - 10:1, preferably of 1:3 - 3:1.

9. The use or medical kit according to any of claims 6 to 8, where the auxiliary material is a contrast material selected from the following group: a substance containing tantalum, iodine, barium, tungsten and/or bismuth.

10. The use or medical kit according to any of claims 6 to 9, where the molecular mass of the polyurethane is 4000 to 70,000 Dalton, preferably 20,000 to 35,000 Dalton.

## Patentansprüche

1. Verwendung eines Polyurethans zur Herstellung einer Komposition zum Ausfüllen oder Kurzschluss von vaskularen Höhlungen, worin die Komposition im Wesen aus in DMSO oder einem C2-C4 Alkohol oder einem Gemisch von diesen gelösten Polyurethan besteht, gegebenenfalls zusammen mit einem oder mehreren Hilfsstoff(en).

2. Komposition zur Verwendung im Ausfüllen oder Kurzschluss von vaskularen Höhlungen, bestehend im Wesen aus in DMSO oder einem C2-C4 Alkohol oder in einem Gemisch von diesen gelöstem Polyurethan, gegebenenfalls zusammen mit einem oder mehreren Hilfsstoff(en).

3. Die Verwendung oder Komposition gemäß Patentanpsruch 1 oder 2, worin das Gemisch von DMSO und EtOH in einem Verhältnis von 1:10 - 10:1, vorzugsweise 1:3 - 3:1 verwendet wird.

4. Verwendung oder Komposition gemäß einem der Patentansprüche 1 bis 3, worin der Hilfsstoff ein Kontrasmaterial gewählt aus der folgenden Gruppe: eine Tantal, Jod, Barium, Wolfram und/oder Bismut enthaltende Substanz ist.

5. Die Verwendung oder Komposition gemäß einem der Ansprüche 1 bis 4, worin die Molekülmasse des Polyurethans 4000 bis 70000 Dalton, vorzugsweise 20 000 bis 35 000 Dalton beträgt.

6. Verwendung eines Polyurethans zur Herstellung einer medizinischen Garnitur zum Ausfüllen oder Kurzschluss von vaskularen Höhlungen, worin die medizinische Garnitur im Wesen aus den folgenden Komponenten besteht, die separat formuliert sind oder manche von diesen in einer gemeinsamen Untereinheit formuliert sind:
a) ein Polyurethan, das in DMSO oder einem C2-C4 Alkohol oder einem Gemisch von diesen lösbar ist,
b) DMSO oder ein C2-C4 Alkohol oder ein Gemisch von diesen,
c) gegebenenfalls ein oder mehrere Hilfsstoff(e).

7. Eine medizinische Garnitur zum Ausfüllen oder Kurzschluss von vaskularen Höhlungen, bestehend im Wesen aus den folgenden Komponenten, die separat formuliert sind oder manche von diesen in einer gemeinsamen Untereinheit formuliert sind:
a) ein Polyurethan, welches in DMSO oder einem C2-C4 Alkohol oder einem Gemisch von diesen lösbar ist,
b) DMSO oder ein C2-C4 Alkohol oder ein Gemisch von diesen,
c) gegebenenfalls ein oder mehrere Hilfsstoff(e).

8. Die Verwendung oder medizinische Garnitur gemäß Patentanspruch 6 oder 7, worin das Gemisch von DMSO und EtOH in einem Volumenverhältnis von 1:10 - 10:1, vorzugsweise 1:3 - 3:1 verwendet wird.

9. Die Verwendung oder medizinische Garnitur gemäß einem der Patentanpsrüche 6 bis 8, worin der Füllstoff ein Kontrastmaterial ist, das aus der folgenden Gruppe gewählt ist: eine Tantal, Jod, Barium, Wolfram und/oder Bismut enthaltende Substanz.

10. Die Verwendung oder medizinische Garnitur gemäß einem der Patentanapsrüche 6 bis 9, worin die Molekülmasse des Polyurethans 4000 bis 70 000 Dalton, vorzugsweise 20 000 bis 35 000 Dalton beträgt.

## Revendications

1. Mise en oeuvre du polyuréthane pour produire une composition servant à remplir ou à court-circuiter des cavités vasculaires, la composition étant constituée essentiellement de polyuréthane dissous dans du DMSO ou dans l'alcool C2-C4 ou bien dans un mélange de ceux-ci, éventuellement en présence d'un ou de plusieurs matériau(x) auxiliaire(s).

2. Une composition à mettre en oeuvre afin de remplir ou de court-circuiter des cavités vasculaires, la composition étant constituée essentiellement de polyuréthane dissous dans du DMSO ou dans l'alcool C2-C4 ou bien dans un mélange de ceux-ci, éventuellement en présence d'un ou de plusieurs matériau(x) auxiliaire(s).

3. Utilisation ou composition selon l'une quelconque des revendications 1 ou 2, où l'on met en oeuvre un mélange de DMSO et d'EtOH en une proportion volumétrique de 1:10 - 10:1, de préférence 1:3 - 3: 1.

4. Utilisation ou composition selon l'une quelconque des revendications 1 à 3, où le matériau auxiliaire est un matériau de contraste choisi dans le groupe suivant : une substance contenant du tantale, de l'iode, du baryum, du tungstène et/ou du bismuth.

5. Utilisation ou composition selon l'une quelconque des revendications 1 à 4, où la masse moléculaire du polyuréthane est de 4000 à 70 000 Daltons, de préférence de 20 000 à 35 000 Daltons.

6. Utilisation d'un polyuréthane pour la préparation d'un kit médical servant à remplir ou à court-circuiter des cavités vasculaires, où le kit médical comprend essentiellement les composants suivants qui sont formulés séparément ou dont quelques uns sont formulés dans une sous-unité commune :
a) un polyuréthane soluble dans le DMSO ou dans l'alcool C2-C4 ou dans un mélange de ceux-ci,
b) du DMSO ou de l'alcool C2-C4 ou un mélange de ceux-ci,
c) éventuellement un ou plusieurs matériau(x) auxiliaire(s).

7. Un kit médical servant à remplir ou à court-circuiter des cavités vasculaires, comprenant essentiellement les composants suivants qui sont formulés séparément ou dont quelques uns sont formulés dans une sous-unité commune :
a) un polyuréthane soluble dans le DMSO ou dans l'alcool C2-C4 ou dans un mélange de ceux-ci,
b) du DMSO ou de l'alcool C2-C4 ou un mélange de ceux-ci,
c) éventuellement un ou plusieurs matériau(x) auxiliaire(s).

8. Mise en oeuvre ou kit médical selon l'une quelconque des revendications 6 ou 7, où un mélange de DMSO et d'EtOH est mis en oeuvre en une proportion volumétrique de 1:10 -10:1, de préférence de 1:3 - 3:1.

9. Mise en oeuvre ou kit médical selon l'une quelconque des revendications 6 à 8, où le matériau auxiliaire est un matériau de contraste choisi dans le groupe suivant : une substance contenant du tantale, de l'iode, du baryum, du tungstène et/ou du bismuth.

10. Mise en oeuvre ou kit médical selon l'une quelconque des revendications 6 à 9, où la masse moléculaire du polyuréthane est de 4000 à 70 000 Daltons, de préférence de 20 000 à 35 000 Daltons.
